(19) Europäisches Patentamt
European Patent Office
Office européen des brevets

(11) Publication number: 0 034 456
B1

(12) # EUROPEAN PATENT SPECIFICATION

(45) Date of publication of patent specification: 15.08.84

(51) Int. Cl.³: C 07 D 413/04, A 01 N 43/80, C 07 D 261/14

(21) Application number: 81300531.1

(22) Date of filing: 10.02.81

(54) 3-(Substituted 3- or 5-isoxazolyl)-1-4-, or 5-substituted-2-imidazolidinones, methods for their preparation, compositions containing them and their use as herbicides.

(30) Priority: 19.02.80 US 122633
18.11.80 US 207151

(43) Date of publication of application:
26.08.81 Bulletin 81/34

(45) Publication of the grant of the patent:
15.08.84 Bulletin 84/33

(84) Designated Contracting States:
AT BE CH DE FR GB IT LI LU NL SE

(56) References cited:
EP - A - 0 002 881
EP - A - 0 044 185
US - A - 4 038 410

(73) Proprietor: PPG INDUSTRIES, INC.
One PPG Place
Pittsburgh Pennsylvania 15272 (US)

(72) Inventor: Lavanish, Jerome Michael
191 Harmon Hills Road
Akron Ohio 44321 (US)

(74) Representative: Taylor, Phillip Kenneth et al,
W.P. THOMPSON & CO. Coopers Building Church Street
Liverpool L1 3AB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 034 456 B1

Courier Press, Leamington Spa, England.

## Description

This invention concerns certain 3-[substituted 3- or 5- isoxazolyl]-1-4-, or 5-substituted-2-imidazolidinones having herbicidal activity, methods for their preparation, and the control of weeds therewith.

This invention provides new compounds having herbicidal properties which are 3-[3- or 5-substituted 3- or 5-isoxazolyl]-1-substituted-4-substituted, 5-substituted, or unsubstituted 2-imidazolidinones represented by the formula:

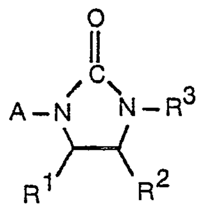

wherein:

A is 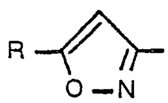 or 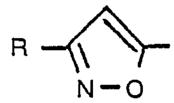

wherein R is alkyl or haloalkyl of up to 6 carbon atoms; cycloalkyl of from 3 to 8 carbon atoms; alkenyl or alkynyl of up to 5 carbon atoms; —$R^4$—O—$R^5$ or —$R^4$—S—$R^5$ wherein $R^4$ is alkylene of up to 6 carbon atoms and $R^5$ is alkyl of up to 6 carbon atoms; or

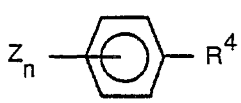 or 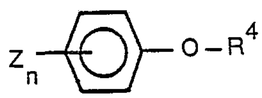

wherein Z is nitro, halogen, trifluoromethyl or $R^5$, and n is 0, 1, 2, or 3;

$R^1$ is hydroxy, halogen or

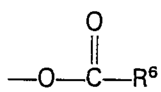

wherein $R^6$ is haloalkyl or up to 9 carbon atoms; cycloalkyl of from 3 to 8 carbon atoms; alkenyl or alkynyl of up to 5 carbon atoms, or

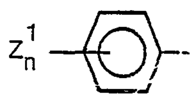

wherein $Z^1$ is nitro, halogen, trifluoromethyl, alkyl or alkoxy of up to 8 carbon atoms; and n is 0, 1, 2, or 3;

$R^2$ is hydrogen, hydroxy, alkyl of up to 4 carbon atoms, or allyl; and

$R^3$ is alkyl of up to 3 carbon atoms or allyl.

EP—A1—0,002,881 describes certain herbicidal 5-isoxazolylurea derivatives of the general formula:—

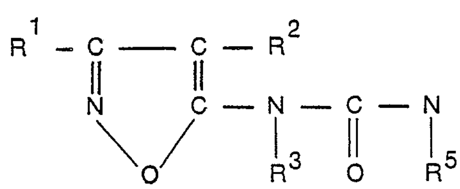

in which $R^3$ and $R^5$ may inter alia together represent optionally substituted alkylene. These compounds differ from those of the present invention in that $R^2$ in the said general formula represents only the

groups cyano, nitro, carboxy, carboxy ester, carboxyamide whose amide group is substituted or a group of the formula

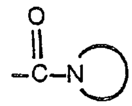

in which

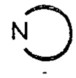

represents a 5—7 membered heterocyclic radical which may optionally be substituted — all of which are highly polar and for the most part functional — and does not represent hydrogen. The presence of such highly polar groups is an essential requirement of the said compounds of European Patent Application No. 0,002,881 and it is therefore surprising that compounds of the present invention in which A is

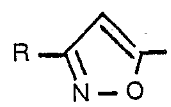

which would correspond to compounds of the said general formula of EP—A1—0,002,881 if $R^2$ in that general formula were hydrogen, possess herbicidal activity.

Some alkyl groups of which the various constituents in the above formula of the present invention are representative are, for example, methyl, ethyl, n-propyl, iso-octyl, nonyl, or the like, including combinations thereof, e.g. dimethylethyl. Exemplary alkoxy groups are methoxy, ethoxy, propoxy, butoxy, octoxy, and the like. As examples of cycloalkyl groups there may be mentioned cyclopropyl, cyclobutyl, cyclopentyl, cyclohexyl, and cyclooctyl. Allyl, butenyl, pentenyl, propynyl, butynyl, pentynyl and the like are exemplary of suitable alkenyl and alkynyl groups represented by the various constituents in the above formula. Representative suitable alkylene groups are, for example, methylene, ethylene, propylene, butylene, pentylene, or hexylene. As halogen substituents, there may be mentioned chlorine, bromine, iodine, or fluorine, preferably chlorine or bromine.

Although any compound within the scope of the above formula of the present invention is believed to have herbicidal activity in accordance with this invention, those compounds that have been found to be especially efficacious are 3 - [5 - (1,1 - dimethylethyl) - 3 - isoxazolyl] - 1 - methyl - 4 - benzoyloxy - 2 - imidazolidinone; 3 - [5 - (1,1 - dimethylethyl) - 3 - isoxazolyl] - 1 - methyl - 4 - hydroxyl - 2 - imidazolidinone; 3 - [3 - (1,1 - dimethylethyl) - 5 - isoxazolyl] - 1 - methyl - 4,5 - dihydroxy - 2 - imidazolidinone; and 3 - [5 - (1,1 - dimethylethyl) - 3 - isoxazolyl] - 1 - methyl - 4,5 - dihydroxy - 2 - imidazolidinone; and 3 - [5 - (1,1 - dimethylethyl) - 3 - isoxazolyl] - 1 - methyl - 4,5 - dihydroxy - 2 - imidazolidinone.

The compounds of this invention wherein $R^1$ is hydroxy and $R^2$ is other than hydroxy may be prepared by reacting a 5-substituted-3-amino isoxazole or a 3-substituted-5-amino isoxazole represented respectively by the formulae:

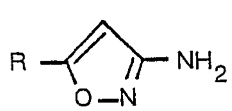 or 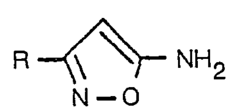

wherein R is as previously defined, with a solvent solution of phosgene and hydrochloric acid to form the corresponding isocyanate. The isocyanate is then reacted with an equivalent amount, preferably in the presence of an inert solvent, of an appropriately substituted amino acetaldehyde dialkylacetal to form an acetal urea of the formula:

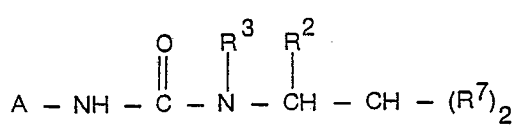

wherein A and $R^3$ are as previously defined, $R^2$ is as previously defined with the exception of hydroxy and $R^7$ is a moiety reactive with the hydrogen atom attached to the nitrogen atom attached to the

isoxazolyl ring represented by A. $R^7$ is preferably alkoxy or alkylthio containing up to 6 carbon atoms. The acetal urea is hydrolyzed in the presence of aqueous hydrochloric acid solution to form a compound of the invention wherein $R^1$ is hydroxy and $R^2$ is other than hydroxy.

Compounds of this invention wherein both $R^1$ and $R^2$ are hydroxy may be prepared by reacting the appropriately substituted aminoisoxazole with an appropriately substituted isocyanate in the presence of an inert solvent and an alkylamine catalyst to form the urea represented by the formula:

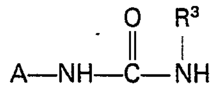

wherein A and $R^3$ are as previously defined. The urea is then reacted with aqueous glyoxal solution (the pH of which solution is adjusted to between 7 and 8) to form compounds of the invention wherein $R^1$ and $R^2$ are hydroxy groups.

Compounds of this invention wherein $R^1$ is

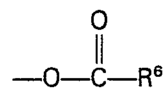

wherein $R^6$ is as previously defined may be prepared by reacting a [3- or 5- isoxazolyl]-1-substituted 4 hydroxy-2-imidazolidone of the formula:

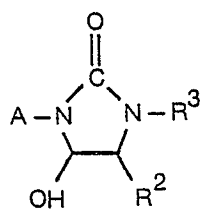

the preparation of which has been previously described with an anhydride of the formula:

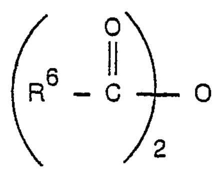

wherein A, $R^2$, $R^3$ and $R^6$ are as previously defined. The reaction is preferably conducted in an inert solvent and in the presence of an acid acceptor such as triethyl amine, pyridine or N,N-dimethylaniline.

Compounds of this invention wherein $R^1$ is halogen, preferably bromine or chlorine, may be prepared by reacting the 3-[3- or 5-substituted 3- or 5-isoxazolyl]-1-substituted-4-hydroxy-2-imidazolidone, the preparation of which has been previously described, with excess halogenating agent such as, for example, thionyl halide, phosphorous oxyhalide, phosphorous trihalide or phosphorous pentahalide, optionally in the presence of an inert organic solvent. An acid binding agent, e.g. an organic base or alkali or alkaline earth metal hydroxides or carbonates, may also be present. When compounds wherein both $R^1$ and $R^2$ are hydroxy are used as a starting material, the hydroxy in the $R^2$ position may be protected by, for example, alkylation followed by subsequent removal of the alkyl group.

To summarize the mode of synthesis described above of compounds of this invention, those compounds wherein $R^1$ or $R^1$ and $R^2$ are hydroxy are first prepared using the described techniques. A compound of this type is then reacted with an anhydride to form compounds wherein $R^1$ is the ester group, —$OCOR^6$; or with a halogenating agent to form compounds wherein $R^1$ is halogen. In other words, certain of the compounds of this invention, i.e. those wherein $R^1$ or $R^1$ and $R^2$ are hydroxy, may also be used as intermediates to prepare other compounds of this invention, i.e. those wherein $R^1$ is halogen or an ester group.

The analytic techniques generally described herein and used to prepare the compounds of this invention are those typically employed by and well-known to those skilled in the art. In addition, the starting materials may be obtained from commercial sources or prepared using known modes of synthesis.

The following examples are illustrative of the preparation of certain specific compounds of this invention.

## Example I

Preparation of 3-[5-(1,1-dimethylethyl)-3-isoxazolyl]-1-methyl-4-hydroxy-2-imidazolidinone

*(a) Formation of 5-(1,1-dimethylethyl)isoxazol-3-yl isocyanate*

A 300 milliliter, 3-neck flask equipped with a magnetic stirrer, thermometer, and dry ice condenser/drying tube was charged with 100 milliliters of ethyl acetate containing 4.8 grams (0.034 mole) of 3-amino-5-(1,1-dimethylethyl)isoxazole. Anhydrous gaseous hydrochloric acid (10.0 grams) was bubbled into the solution, and then 20 grams of gaseous phosgene was bubbled into the solution, which was cooled in an ice bath. The solution was allowed to stand at ambient temperature for 17 hours and then the flask was purged with argon until no phosgene was detected. The solution was filtered under nitrogen, and the precipitate was washed with benzene yielding 5.6 grams (0.034 mole) of 5-(1,1-dimethylethyl)isoxazol-3-yl isocyanate.

*(b) Formation of 3-[5-(1,1-dimethylethyl)-3-isoxazolyl]-1-methyl-1-(2,2-dimethoxyethyl)urea*

At ambient temperature 4.2 grams (0.035 mole) of methylaminoacetaldehyde dimethylacetal in 15 milliliters of benzene was rapidly added to 50 milliliters of benzene containing 0.034 mole (5.6 grams) of the 5-(1,1-dimethylethyl)isoxazolyl-3-yl isocyanate prepared in step (a). The resulting slurry was heated to reflux for two (2) minutes, filtered, cooled, and 20 milliliters of hexane added. No crystals formed upon standing and cooling in a refrigerator. The slurry was then concentrated on a rotary evaporator at 70°C. to yield 5.7 grams of a viscous oil containing 3 - [5 - (1,1 - dimethylethyl) - 3 - isoxazolyl] - 1 - methyl - 1 - (2,2 - dimethoxyethyl)urea. The oil crystallized on cooling, and the crystals were recrystallized from ethyl ether/hexane solution upon refrigeration. The crystals were removed by suction filtration and air dried to yield 5.2 grams of white crystals of 3 - [5 - (1,1 - dimethylethyl) - 3 - isoxazolyl] - 1 - methyl - 1 - (2,2 - dimethoxyethyl)urea. M.P. 80—84°C., IR spectra (mull.) bands at 3260, 1670, 1600, and 1530 $cm^{-1}$, MS ion at m/e at 285.

NMR ($CDCl_3$) 9.11 $\delta$ (singlet, 1H); 6.61 $\delta$ (singlet, 1H);
4.52 $\delta$ (triplet, 1H); 3.42 $\delta$ (singlet, 8H);
3.50 $\delta$ (doublet, 8H); 3.13 $\delta$ (singlet, 3H);
1.30 $\delta$ (singlet, 9H).

*(c) Preparation of 3-[5-(1,1-dimethylethyl)-3-isoxazolyl]-1-methyl-4-hydroxy-2-imidazolidinone*

A round bottom flask was charged with 2.6 grams of the 3[5 - (1,1 - dimethylethyl) - 3 - isoxazolyl] - 1 - methyl-(2,2 - dimethoxyethyl)urea prepared in step (b), 150 milliliters of water and 1.5 milliliters of concentrated hydrochloric acid. The resulting mixture was heated until one phase formed and crystals coated out on the sides of the flask and cooled; and the scrapings of the crystals and the solution were filtered. The crystalline precipitate was washed twice with separate portions of water, and the air dried to yield 1.9 grams of white crystals of 3 - [5 - (1,1 - dimethylethyl) - 3 - isoxazolyl] - 1 - methyl - 4 - hydroxy - 2 - imidazolidinone. M.P. 173—177°C., IR spectra (mull.) bands at 3400, 3140, 1700 and 1590 $cm^{-1}$.

NMR ($CDCl_3$): 6.64 $\delta$ (singlet, 1H); 5.83 $\delta$ (multiplet, 1H);
4.92 $\delta$ (broad singlet, 1H); $\delta$ 3.83—3.17 (multiplet, 2H);
2.89 $\delta$ (singlet, 3H); 1.30 $\delta$ (singlet, 9H).

## Example II

Preparation of 3-[3-(1,1 -dimethylethyl)-5-isoxazolyl]-4,5-dihydroxy-1-methyl-2-imidazolidinone

*(a) Formation of 1-[3-(1,1-dimethylethyl)-5-isoxazolyl]-3-methylurea*

A 50 milliliter flask provided with a magnetic stirring bar was charged with 1.3 grams (0.0093 mole) of 3-(1,1-dimethylethyl)-5-isoxazolamine and 25 milliliters of benzene. To this mixture was added 1.7 grams of methyl isocyanate and one (1) drop of triethylamine; and the mixture was allowed to stand overnight, after which it was heated to reflux for 7.5 hours and then allowed to stand at room temperature for two (2) days. Thin layer chromatography showed only partial reaction so a trace of 4-dimethylaminopyridine and several milliliters of methyl isocyanate were added and the mixture heated at reflux for 4.5 hours. The mixture was then concentrated on a rotary evaporator to give 2.6 grams of a viscous red brown oil. Chromatography on alumina with chloroform/ethyl acetate monitored by thin layer chromatography gave fractions containing a single component. These fractions were combined and evaporated to give 0.67 gram of a pale yellow solid of 1 - [3 - (1,1 - dimethylethyl) - 5 - isoxazolyl] - 3 - methylurea, M.P. 188—196°C., which showed a molecular ion at 197 in the mass spectrum.

*(b) Formation of 3-[3-(1,1-dimethylethyl)-5-isoxazolyl]-4,5-dihydroxy-1-methyl-2-imidazolidinone*

The 0.67 gram (0.0034 mole) of 1 - [3 - (1,1 - dimethylethyl) - 5 - isoxazolyl] - 3 - methylurea prepared in step (a) was dissolved in 5 milliliters of ethanol. To this solution was added 1.5 grams of a 40 percent aqueous glyoxal solution previously adjusted to pH 7 with dilute aqueous sodium hydroxide solution. After standing at room temperature for 2.5 days, the mixture was concentrated on a rotary evaporator and the residue was washed with water and extracted with chloroform. The organic

extract was dried over magnesium sulfate, filtered, and concentrated on a rotary evaporator to give 0.9 gram of viscous residue. Chromatography on alumina with ethylacetate/ethanol gave fractions containing a single component as monitored by thin layer chromatography. These fractions were combined and concentrated on a rotary evaporator to give 0.16 gram of oily residue which partially crystallized on cooling. Recrystallization from diethylether gave 0.08 gram of white crystals of 3 - [3 - (1,1 - dimethylethyl) - 5 - isoxazolyl] - 4,5 - dihydroxy - methyl - 2 - imidazolidinone, M.P. 153—156°C., which had mass, infrared and NMR spectra consistent with the desired product.

Example III

Preparation of 3-[5-(1,1-dimethylethyl)-3-isoxazolyl]-4,5-dihydroxy-1-methyl-2-imidazolidinone

*(a) Formation of 1-[5-(1,1-dimethylethyl-3-isoxazolyl]-3-methylurea*

A 50 milliliter flask provided with a magnetic stirring bar, addition funnel, thermometer and condenser/drying tube was charged with 3.0 grams (0.021 mole) of 5-(1,1-dimethylethyl)-3-isoxazolamine, a few crystals of 4-dimethylaminopyridine and 20 milliliters of benzene. The funnel was charged with 2.3 grams (0.040 mole) of methyl isocyanate in 5 milliliters of benzene, which was added dropwise over five (5) minutes. After standing overnight, the mass of crystals which formed was isolated by filtration, washed with hexane, and air dried to give 3.0 grams of white powder of 1 - [5 - (1,1 - dimethylethyl) - 3 - isoxazolyl] - 3 - methylurea, M.P. 188—189°C.

*(b) Formation of 3-[5-(1,1-dimethylethyl)-3-isoxazolyl]-4,5-dihydroxy-1-methyl-2-imidazolidinone*

To a solution of 2.0 grams (0.010 mole) of 1 - [5(1,1 - dimethylethyl) - 3 - isoxazolyl] - 3 - methylurea, prepared in step (a), in 50 milliliters of 95 percent ethanol was added 7.3 grams of a 40 percent aqueous glyoxal solution which had been previously adjusted to pH 7 with dilute sodium hydroxide solution. After standing at room temperature overnight, the solution was concentrated on a rotary evaporator to give an oily residue which was extracted with two 20 milliliter portions of chloroform. The extract was washed with saturated sodium chloride solution, filtered through silicone-treated paper, and concentrated on a rotary evaporator to give 2.0 grams of oily residue. Crystallization from ether gave 0.4 gram of white crystals of 3 - [5 - (1,1 - dimethylethyl) - 3 - isoxazolyl] - 4,5 - dihydroxy - 1 - methyl - 2 - imidazolidinone, M.P. 153—157°C., which had consistent mass, infrared and NMR spectra.

Example IV

Preparation of 3-[5-(1,1-dimethylethyl)-3-isoxazolyl]-1-methyl-4-benzoyloxy-2-imidazolidinone

A 50-milliliter flask provided with a reflux condenser, drying tube and magnetic stirring bar was charged with 1.0 grams of 3 - [5 - (1,1 - dimethylethyl) - 3 - isoxazolyl] - 1 - methyl - 4 - hydroxy - 2 - imidazolidone (prepared as described in Example I), 1.1 grams of benzoic anhydride, 0.5 gram of triethylamine and 20 milliliters of benzene. The flask contents were warmed slightly until the solution clarified and allowed to stand overnight with stirring. Thin layer chromatography indicated that the reaction was not complete and an additional 1.0 gram of benzoic anhydride and 0.5 gram of triethylamine were added. The reaction mixture was then heated to reflux and maintained at reflux for about 3.5 hours. After refluxing, the reaction mixture was permitted to stand at ambient temperature for 2.5 days. The reaction mixture was then transferred to a separatory funnel and washed consecutively with 50 milliliter aliquots of water, 5 percent aqueous hydrochloric acid solution, 5 percent aqueous sodium bicarbonate solution, 5 percent aqueous sodium bicarbonate solution, and saturated brine. The organic phase was separated, dried over magnesium sulfate, filtered, and concentrated on a rotary evaporator at 55°C., yielding 1.9 grams of a partially crystalline residue. The residue was recrystallized from a mixture of benzene and hexane yielding 0.8 gram of white crystals having a melting point of 165—166°C. and identified by mass spectrum analysis as 3 - [5 - (1,1 - dimethylethyl) - 3 - isoxazolyl] - 1 - methyl - 4 - benzoyloxy - 2 - imidazolidinone.

The mode of synthesis of specific compounds of this invention have been illustrated by the foregoing Examples; but it is to be understood that any compound contemplated within the scope of this invention may be prepared by those skilled in the art simply by varying the choice of starting materials and using the illustrated techniques or other suitable techniques.

The compounds of this invention are effective in regulating the growth of a variety of undesirable plants, i.e. weeds, when applied, in an herbicidally effective amount, to the growth medium prior to emergence of the weeds or to the weeds subsequent to emergence from the growth medium. In addition, the compounds of this invention have been found to be herbicidally active when applied using the preplant incorporation technique, wherein the compound is incorporated into the soil prior to crop planting. The term "herbicidally effective amount" is that amount of compound or mixture of compounds required to so injure or damage weeds such that the weeds are incapable of recovering following application. The quantity of a particular compound or mixture of compounds applied in order to exhibit a satisfactory herbicidal effect may vary over a wide range and depends on a variety of factors such as, for example, hardiness of a particular weed species, extent of weed infestation, climatic conditions, soil conditions, method of application, and the like. Typically, as little as 0.2 (90.72) or less pound (g) per acre to 10 (4.536) or more pounds (kg) per acre (4047 $m^2$) of compound or mixtures of

compounds may be required. Of course, the efficacy of a particular compound against a particular weed species may readily be determined by relatively straightforward laboratory or field testing in a manner well known to the art.

The compounds of this invention may be used as such or in formulation with agronomically acceptable adjuvants, inert carriers, other herbicides, or other commonly used agricultural compounds, for example, pesticides, stabilizers, safeners, fertilizers. The compounds of this invention, whether or not in formulation with other agronomically acceptable materials, are typically applied in the form of dusts, granules, wettable powders, solutions, suspension, aerosols, emulsions, dispersions in a manner well known in the art. When formulated with other typically used agronomically acceptable materials, the amount of compound or compounds of this invention present in the formulation may vary over a wide range, for example, from 0.05 to 95 percent by weight on weight of formulation. Typically such formulations will contain from 5 to 75 percent by weight of compound or compounds of this invention.

The compounds of this invention as exemplified by the compounds prepared in Examples I through VI have been found effective in controlling a variety of broadleaf and grassy weeds at application rates of as little as 0.25 pound (0.1134 kg) per acre (4047 $m^2$), preemergence or postemergence. The compounds prepared according to Examples I through VI were tested for herbicidal activity against various weed species under controlled laboratory conditions of light, temperature, and humidity, using techniques known to the art. In preemergence evaluation, a solvent solution of the test compound is applied at the desired rate to the weed species prior to emergence from the growth medium whereas in postemergent evaluation a solvent solution of the test compound is applied at the desired rate directly on the growing plant, the toxic effect of the compound being determined by visual inspection periodically after application.

Each of the compounds prepared in Examples I through VI were individually applied both preemergence and postemergence at application rates ranging from 0.25 to 1.0 pound per acres (0.1134 to 0.4536 kg per 4047 $m^2$) to broadleaf weeds, namely teaweed, jimsonweed, wild mustard, coffeeweed, velvetleaf, and morningglory and to grassy weeds, namely, yellow foxtail, crabgrass, johnsongrass, wild oats, and barnyardgrass. Within 21 days of application of each compound, each of the weed species was either killed or injured beyond recovery.

**Claims for the Contracting States: BE CH DE FR GB IT LI LU NL SE**

1. A compound characterised by the formula:

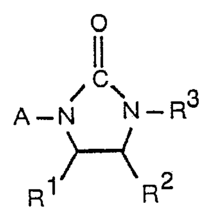

wherein:

A is 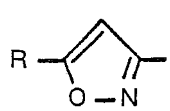 or 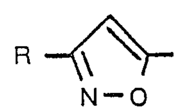

wherein R is alkyl or haloalkyl of up to 6 carbon atoms; cycloalkyl of from 3 to 8 carbon atoms; alkenyl or alkynyl of up to 5 carbon atoms; —$R^4$—O—$R^5$ or —$R^4$—S—$R^5$ wherein $R^4$ is alkylene of up to 6 carbon atoms and $R^5$ is alkyl of up to 6 carbon atoms; or

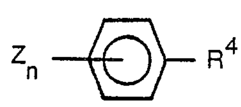 or 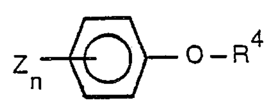

wherein Z is nitro, halogen, trifluoromethyl or $R^5$, and n is 0, 1, 2, or 3; $R^1$ is hydroxy, halogen or

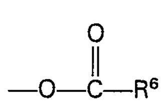

wherein $R^6$ is haloalkyl of up to 9 carbon atoms; cycloalkyl of from 3 to 8 carbon atoms; alkenyl or alkynyl of up to 5 carbon atoms, or

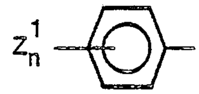

wherein $Z^1$ is nitro, halogen, trifluoromethyl, alkyl or alkoxy of up to 8 carbon atoms; and n is 0, 1, 2, or 3; $R^2$ is hydrogen, hydroxy, alkyl of up to 4 carbon atoms, allyl; and $R^3$ is alkyl of up to 3 carbon atoms or allyl.

2. A compound of claim 1 wherein R and $R^3$ are alkyl, $R^1$ is hydroxy and $R^2$ is hydrogen or hydroxy.

3. A compound of claim 2 selected from 3 - [5 - (1,1 - dimethylethyl) - 3 - isoxazolyl] - 1 - methyl - 4 - hydroxy - 2 - imidazolidinone, 3 - [3 - (1,1 - dimethylethyl) - 5 - isoxazolyl] - 1 - methyl - 4,5 - dihydroxy - 2 - imidazolidinone, or 3 - [5 - (1,1 - dimethylethyl) - 3 - isoxazolyl] - 1 - methyl - 4,5 - dihydroxy - 2 - imidazolidone.

4. A compound of claim 1 wherein R and $R^3$ are alkyl, $R^2$ is hydrogen and $R^1$ is

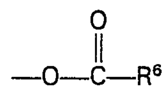

wherein $R^6$ is phenyl.

5. 3 - [5 - (1,1 - dimethylethyl) - 3 - isoxazolyl] - 1 - methyl - 4 - benzoyloxy - 2 - imidazolidinone.

6. A herbicidal composition containing a herbicidally effective amount of a compound or mixture of compounds defined in claim 1.

7. A method of controlling weed growth wherein a herbicidally effective amount of a herbicide is applied to a growth medium prior to emergence of weeds from or applied to the weeds subsequent to emergence from the growth medium, characterised in that the herbicide is a compound or mixture of compounds defined in claim 1.

8. A method for preparing a compound claimed in claim 1 characterised by:

(a) phosgenating an appropriately substituted amino isoxazole represented by the formulae:

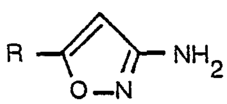 or 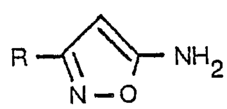

wherein R is as defined in claim 1 to form the corresponding isocyanate;

(b) reacting the isocyanate with an appropriately substituted amino acetaldehyde dialkylacetal to form an acetal urea of the formula:

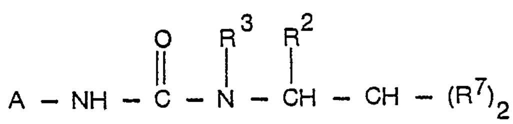

wherein A and $R^3$ are as defined in claim 1, $R^2$ is as defined in claim 1 with the exception of hydroxy and $R^7$ is alkoxy or alkylthio containing up to 6 carbon atoms; and

(c) hydrolyzing the acetal urea, to form a compound as claimed in claim 1 wherein $R^1$ is hydroxy and $R^2$ is other than hydroxy.

9. The method of claim 8 characterised by the further step of either reacting the hydrolysis product of step (c) with a halogenating agent to form a compound claimed in claim 1 wherein $R^1$ is halogen; or reacting the hydrolysis product of step (c) with an anhydride of the formula:

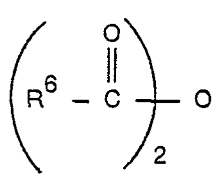

wherein $R^6$ is as previously defined to form a compound claimed in claim 1 wherein $R^1$ is

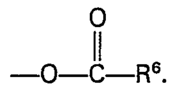

10. A method of preparing a compound claimed in claim 1 characterised by:
(a) reacting an appropriately substituted aminoisoxazole represented by the formulae:

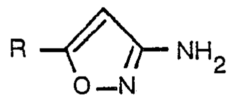 or 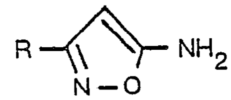

wherein R is as defined in claim 1 with an appropriately substituted isocyanate to form a urea of the formula:

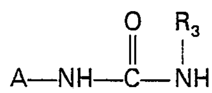

wherein A and $R^3$ are as defined in claim 1; and
(b) reacting the urea with aqueous glyoxal solution to form a compound claimed in claim 1 wherein $R^1$ and $R^2$ are both hydroxy.

11. The method of claim 10 characterised by the further step of either reacting the reaction product of step (b) with a halogenating agent to form a compound claimed in claim 1 wherein $R^1$ is halogen or reacting the reaction product of step (b) with an anhydride of the formula:

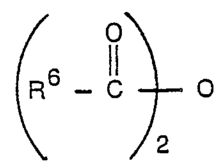

wherein $R^6$ is as defined in claim 1 to form a compound claimed in claim 1 wherein $R^1$ is

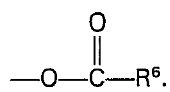

**Claims for the Contracting State: AT**

1. A method of preparing a compound having the formula:

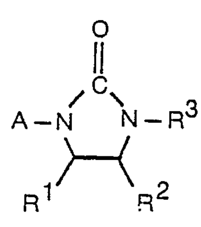 I

wherein:

A is 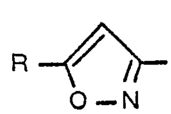 or 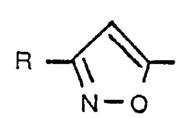

wherein R is alkyl or haloalkyl of up to 6 carbon atoms; cycloalkyl of from 3 to 8 carbon atoms; alkenyl or alkynyl of up to to 5 carbon atoms; —$R^4$—O—$R^5$ or —$R^4$—S—$R^5$ wherein $R^4$ is alkylene of up to 6 carbon atoms and $R^5$ is alkyl of up to 6 carbon atoms; or

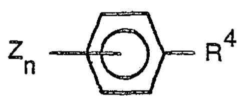 or 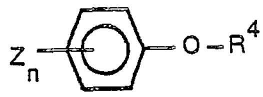

wherein Z is nitro, halogen, trifluoromethyl or $R^5$, and n is 0, 1, 2, or 3; $R^1$ is hydroxy, halogen or

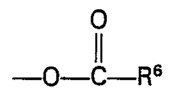

wherein $R^6$ is haloalkyl of up to 9 carbon atoms; cycloalkyl of from 3 to 8 carbon atoms; alkenyl or alkynyl of up to 5 carbon atoms, or

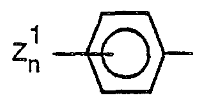

wherein $Z^1$ is nitro, halogen, trifluoromethyl, alkyl or alkoxy of up to 8 carbon atoms; and n is 0, 1, 2, or 3; $R^2$ is hydrogen, hydroxy, alkyl of up to 4 carbon atoms, allyl; and $R^3$ is alkyl of up to 3 carbon atoms or allyl, characterised by:—

(a) phosgenating an appropriately substituted amino isoxazole represented by the formulae:

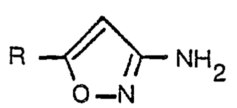 or 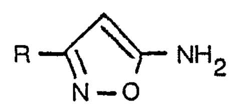 II

wherein R is as defined above to form the corresponding isocyanate;

(b) reacting the isocyanate with an appropriately substituted amino acetaldehyde dialkylacetal to form an acetal urea of the formula:

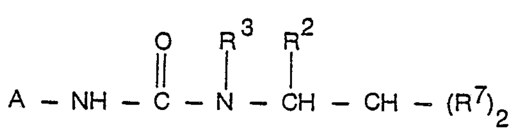 III

wherein A and $R^3$ are as defined above, $R^2$ is as defined above with the exception of hydroxy and $R^7$ is alkoxy or alkylthio containing up to 6 carbon atoms; and

(c) hydrolyzing the acetal urea, to form a compound of the formula I above wherein $R^1$ is hydroxy and $R^2$ is other than hydroxy.

2. The method of claim 1 characterised by the further step of either reacting the hydrolysis product of step (c) with a halogenating agent to form a compound of formula I wherein $R^1$ is halogen; or reacting the hydrolysis product of step (c) with an anhydride of the formula:

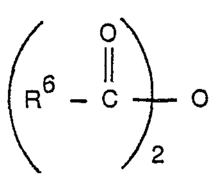 IV

wherein $R^6$ is as previously defined to form a compound of the formula I wherein $R^1$ is

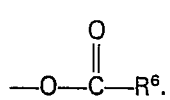

3. A method of preparing a compound of the formula I defined in claim 1 characterised by: (a) reacting an appropriately substituted aminoisoxazole represented by the formulae:

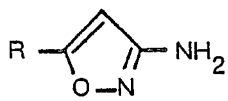 or 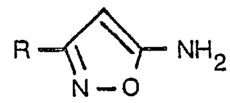

wherein R is as defined in claim 1 with an appropriately substituted isocyanate to form a urea of the formula:

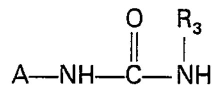

wherein A and $R^3$ are as defined in claim 1; and

(b) reacting the urea with aqueous glyoxal solution to form a compound of the formula I defined in claim 1 wherein $R^1$ and $R^2$ are both hydroxy.

4. The method of claim 3 characterised by the further step of either reacting the reaction product of step (b) with a halogenating agent to form a compound of the formula I defined in claim 1 wherein $R^1$ is halogen or reacting the reaction product of step (b) with an anhydride of the formula:

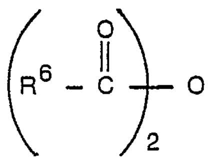

wherein $R^6$ is as defined in claim 1 to form a compound of the formula I defined in claim 1 wherein $R^1$ is

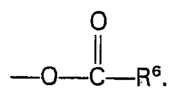

5. A method as claimed in claim 1 characterised in that R and $R^3$ are alkyl, $R^1$ is hydroxy and $R^2$ is hydrogen.

6. A method as claimed in claim 3 characterised in that R and $R^3$ are alkyl.

7. A method as claimed in claim 2 characterised in that R and $R^3$ are alkyl, $R^2$ is hydrogen and $R^1$ is

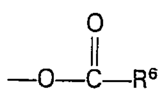

wherein $R^6$ is phenyl.

8. A method of controlling weed growth wherein a herbicidally effective amount of a herbicide is applied to a growth medium prior to emergence of weeds from, or applied to weeds subsequent to emergence from the growth medium, characterised in that the herbicide is a compound or a mixture of compounds of the formula I defined in claim 1.

**Patentansprüche für die Vertragsstaaten: BE CH DE FR GB IT LI LU NL SE**

1. Verbindung, gekennzeichnet durch die Formel

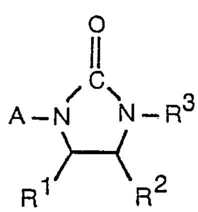

worin bedeuten

A is 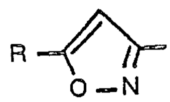 oder 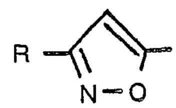

worin R Alkyl oder Halogenalkyl mit bis zu 6 C-Atomen ist;
Cycloalkyl mit 3 bis 8 C-Atomen;
Alkenyl oder Alkinyl mit bis zu 5 C-Atomen;
—$R^4$—O—$R^5$ oder —$R^4$—S—$R^5$, worin $R^4$ Alkylen mit bis zu 6 C-Atomen und $R^5$ Alkyl mit bis zu 6 C-Atomen ist;
oder

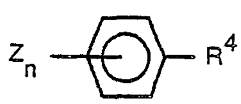 oder 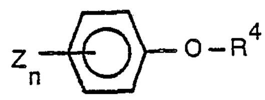

worin Z Nitro, Halogen, Trifluormethyl oder $R^5$ ist und n gleich 0, 1, 2 oder 3 ist;
$R^1$ Hydroxy, Halogen oder

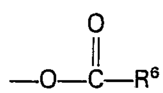

ist, worin $R^6$ Halogenalkyl mit bis zu 9 C-Atomen ist;
Cycloalkyl mit 3 bis 8 C-Atomen;
Alkenyl oder Alkinyl mit bis zu 5 C-Atomen; oder

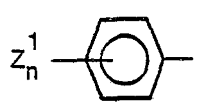

worin $Z^1$ Nitro, Halogen, Trifluormethyl, Alkyl oder Alkoxy mit bis zu 8 C-Atomen ist und n gleich 0, 1, 2 oder 3 ist;
$R^2$ Wasserstoff, Hydroxy, Alkyl mit bis zu 4 C-Atomen, Allyl; und
$R^3$ Alkyl mit bis zu 3 C-Atomen oder Allyl.

2. Verbindung nach Anspruch 1, worin R und $R^3$ Alkyl sind, $R^1$ Hydroxy ist und $R^2$ Wasserstoff oder Hydroxy ist.

3. Verbindung nach Anspruch 2, ausgewählt aus 3 - [5 - (1,1 - Dimethyläthyl) - 3 - isoxazolyl] - 1 - methyl - 4 - hydroxy - 2 - imidazolidinon, 3 - [3 - (1,1 - Dimethyläthyl) - 5 - isoxazolyl] - 1 - methyl - 4,5 - dihydroxy - 2 - imidazolidinon oder 3 - [5 - (1,1 - Dimethyläthyl) - 3 - isoxazolyl] - 1 - methyl - 4,5 - dihydroxy - 2 - imidazolidinon.

4. Verbindung nach Anspruch 1, worin R und $R^3$ Alkyl sind, $R^2$ Wasserstoff ist und $R^1$

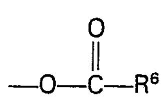

ist, worin $R^6$ Phenyl ist.

5. 3-[5-(1,1-Dimethyläthyl)-3-isoxazolyl]-1-methyl-4-benzoyloxy-2-imidazolidinon.

6. Herbizide Zusammensetzung, enthaltend eine herbizid wirksame Menge einer Verbindung oder eines Gemisches von Verbindungen, wie im Anspruch 1 definiert.

7. Verfahren zum Steuern des Wuchses von Unkraut, worin eine herbizid wirksame Menge eines Herbizids auf ein Wuchsmedium vor dem Sprießen von Unkraut aufgebracht wird oder auf das Unkraut nach dessen Sprießen aus dem Wuchsmedium aufgebracht wird, dadurch gekennzeichnet, daß das Herbizid eine Verbindung oder ein Gemisch von Verbindungen, wie im Anspruch 1 definiert, ist.

8. Verfahren zur Herstellung einer Verbindung nach Anspruch 1, dadurch gekennzeichnet, daß (a) ein entsprechend substituiertes Aminoisoxazol der Formeln

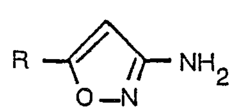 oder 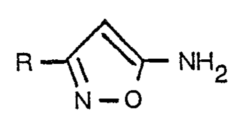

worin R wie im Anspruch 1 definiert ist, phosgeniert wird, um das entsprechende Isocyanat zu bilden; (b) das Isocyanat mit einem entsprechend substituierten Aminoacetaldehyddialkylacetal umgesetzt wird, um einen Acetalharnstoff der Formel

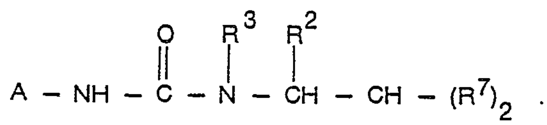

zu bilden, worin A und $R^3$ wie im Anspruch 1 definiert sind, $R^2$ wie im Anspruch 1 definiert ist, mit der Ausnahme von Hydroxy, und $R^7$ Alkoxy oder Alkylthio mit bis zu 6 C-Atomen ist; und
(c) der Acetalharnstoff hydrolysiert wird, um einer Verbindung wie im Anspruch 1 zu bilden, worin $R^1$ Hydroxy ist und $R^2$ von Hydroxy verschieden ist.

9. Verfahren nach Anspruch 8, dadurch gekennzeichnet, daß in einer weiteren Stufe entweder das Hydrolysenprodukt aus Stufe (c) mit einem Halogenierungsmittel umgesetzt wird, um eine Verbindung, wie im Anspruch 1 definiert, zu erhalten, worin $R^1$ Halogen ist; oder das Hydrolysenprodukt aus Stufe (c) mit einem Anhydrid der Formel

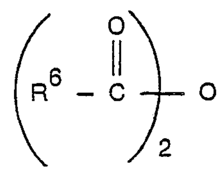

umgesetzt wird, worin $R^6$ wie vorher definiert ist, um eine Verbindung, wie im Anspruch 1 definiert, zu bilden, worin $R^1$

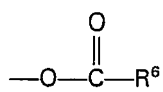

ist.

10. Verfahren zur Herstellung einer Verbindung nach Anspruch 1, dadurch gekennzeichnet, daß (a) ein entsprechend substituiertes Aminoisoxazol der Formeln

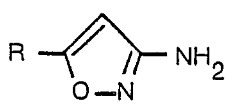
oder
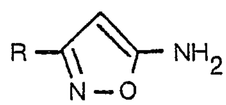

worin R wie im Anspruch 1 definiert ist, mit einem entsprechend substituierten Isocyanat umgesetzt wird, um einen Harnstoff der Formel

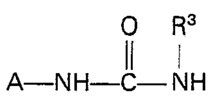

zu bilden, worin A und $R^3$ wie im Anspruch 1 definiert sind; und
(b) der Harnstoff mit einer wässerigen Glyoxallösung umgesetzt wird, um eine Verbindung, wie im Anspruch 1 definiert, zu bilden, worin $R^1$ und $R^2$ beide Hydroxy sind.

11. Verfahren nach Anspruch 10, dadurch gekennzeichnet, daß in einer weiteren Stufe entweder das Reaktionsprodukt aus Stufe (b) mit einem Halogenierungsmittel umgesetzt wird, um eine Verbindung, wie im Anspruch 1 definiert, zu bilden, worin $R^1$ Halogen ist; oder das Reaktionsprodukt aus Stufe (b) mit einem Anhydrid der Formel

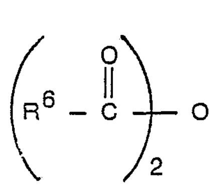

umgesetzt wird, worin $R^6$ wie im Anspruch 1 definiert ist, um eine Verbindung, wie im Anspruch 1 definiert, zu bilden, worin $R^1$

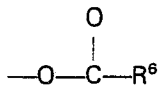 ist.

**Patentansprüche für den Vertragsstaat: AT**

1. Verfahren zur Herstellung einer Verbindung der Formel

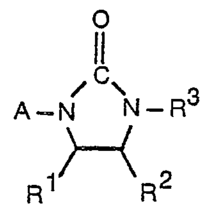

worin bedeuten:

A is 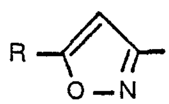 oder 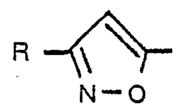

worin R Alkyl oder Halogenalkyl mit bis zu 6 C-Atomen ist;
Cycloalkyl mit 3 bis 8 C-Atomen;
Alkenyl oder Alkinyl mit bis zu 5 C-Atomen;
—$R^4$—O—$R^5$ oder —$R^4$—S—$R^5$, worin $R^4$ Alkylen mit bis zu 6 C-Atomen und $R^5$ Alkyl mit bis zu 6 C-Atomen ist; oder

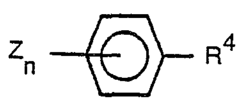 oder 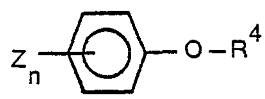

worin Z Nitro, Halogen, Trifluormethyl oder $R^5$ ist und n gleich 0, 1, 2 oder 3 ist;
$R^1$ Hydroxy, Halogen oder

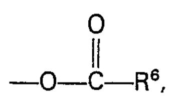,

worin $R^6$ Halogenalkyl mit bis zu 9 C-Atomen ist;
Cycloalkyl mit 3 bis 8 C-Atomen;
Alkenyl oder Alkinyl mit bis zu 5 C-Atomen; oder

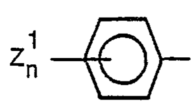

worin $Z^1$ Nitro, Halogen, Trifluormethyl, Alkyl oder Alkoxy mit bis zu 8 C-Atomen ist und n gleich 0, 1, 2 oder 3 ist;
$R^2$ Wasserstoff, Hydroxy, Alkyl mit bis zu 4 C-Atomen, Allyl; und
$R^3$ Alkyl mit bis zu 3 C-Atomen oder Allyl, dadurch gekennzeichnet, daß
(a) ein entsprechend substituiertes Aminoisoxazol der Formeln

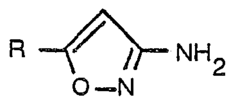 oder 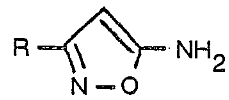

worin R wie vorstehend definiert ist, phosgeniert wird, um das entsprechende Isocyanat zu bilden; (b) das Isocyanat mit einem entsprechend substituierten Aminoacetaldehyddialkylacetal umgesetzt wird, um einen Acetylharnstoff der Formel

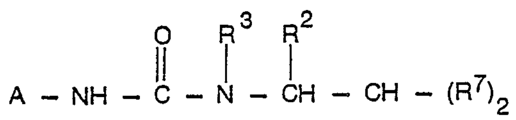 (III)

zu bilden, worin A und $R^3$ wie vorstehend definiert sind, $R^2$ wie vorstehend definiert ist, mit der Ausnahme von Hydroxy, und $R^7$ Alkoxy oder Alkylthio mit bis zu 6 C-Atomen ist; und (c) der Acetalharnstoff hydrolysiert wird, um eine Verbindung der Formel (I) zu bilden, worin $R^1$ Hydroxy ist und $R^2$ von Hydroxy verschieden ist.

2. Verfahren nach Anspruch 1, dadurch gekennzeichnet, daß in einer weiteren Stufe entweder das Hydrolysenprodukt aus Stufe (c) mit einem Halogenierungsmittel umgesetzt wird, um eine Verbindung der Formel (I) zu erhalten, worin $R^1$ Halogen ist; oder das Hydrolysenprodukt aus Stufe (c) mit einem Anhydrid der Formel

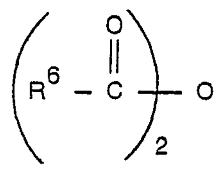 (IV)

umgesetzt wird, worin $R^6$ wie vorher definiert ist, um eine Verbindung der Formel (I) zu bilden, worin $R^1$ gleich ist

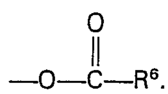

3. Verfahren zur Herstellung einer Verbindung der Formel (I), wie im Anspruch 1 definiert, dadurch gekennzeichnet, daß
(a) ein entsprechend substituiertes Aminoisoxazol der Formeln

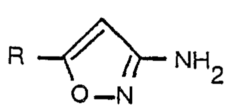 oder 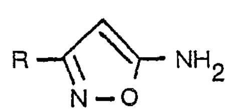

worin R wie im Anspruch 1 definiert ist, mit einem entsprechend substituierten Isocyanat umgesetzt wird, um einen Harnstoff der Formel

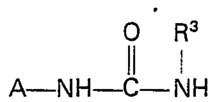

zu bilden, worin A und $R^3$ wie im Anspruch 1 definiert sind; und
(b) der Harnstoff mit einer wässerigen Glyoxallösung umgesetzt wird, um eine Verbindung der Formel (I), wie im Anspruch 1 definiert, zu bilden, worin $R^1$ und $R^2$ beide Hydroxy sind.

4. Verfahren nach Anspruch 3, dadurch gekennzeichnet, daß in einer weriteren Stufe entweder das Reaktionsprodukt aus Stufe (b) mit einem Halogenierungsmittel umgesetzt wird, um eine Verbindung der Formel (I), wie im Anspruch 1 definiert, zu bilden, worin $R^1$ Halogen ist; oder das Reaktionsprodukt aus Stufe (b) mit einem Anhydrid der Formel

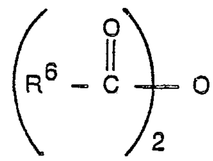

umgesetzt wird, worin $R^6$ wie im Anpruch 1 definiert ist, um eine Verbindung der Formel (I), wie im Anspruch 1 definiert, zu bilden, worin $R^1$ gleich ist

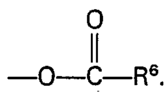

5. Verfahren nach Anspruch 1, dadurch gekennzeichnet, daß R und $R^3$ Alkyl sind, $R^1$ Hydroxy ist und $R^2$ Wasserstoff ist.

6. Verfahren nach Anspruch 3, dadurch gekennzeichnet, daß R und $R^3$ Alkyl sind.

7. Verfahren nach Anspruch 2, dadurch gekennzeichnet, daß R und $R^3$ Alkyl sind, $R^2$ Wasserstoff ist und $R^1$ gleich ist

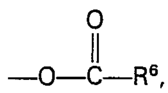

worin $R^6$ Phenyl ist.

8. Verfahren zur Steuern des Wuchses von Unkraut, worin eine herbizid wirksame Menge eines Herbizids auf ein Wuchsmedium vor dem Sprießen von Unkraut aufgebracht wird oder auf das Unkraut nach dessen Sprießen aus dem Wuchsmedium aufgebracht wird, dadurch gekennzeichnet, daß das Herbizid eine Verbindung oder ein Gemisch von Verbindungen der Formel (I), wie im Anspruch 1 definiert, ist.

**Revendications pour les Etats contractants: BE CH DE FR GB IT LI LU NL SE**

1. Un composé caractérisé par la formule:

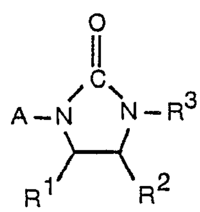

dans laquelle

A est 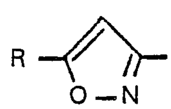 ou 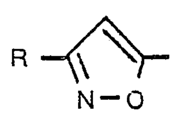

où R est un alkyle ou halogénoalkyle ayant jusqu'à 6 atomes de carbone; un cycloalkyle de 3 à 8 atomes de carbone; un alcényle ou un alcynyle ayant jusqu'à 5 atomes de carbone; —$R^4$—O—$R^5$ ou —$R^4$—S—$R^5$ où $R^4$ est un alkylène ayant jusqu'à 6 atomes de carbone et $R^5$ est un alkyle ayant jusqu'à 6 atomes de carbone; ou

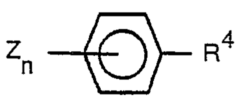 ou 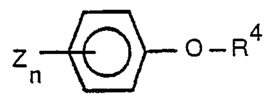

ou Z est un nitro, un halogène, un trifluorométhyle ou $R^5$, et n est 0, 1, 2 ou 3; $R^1$ est un hydroxy, un halogène ou

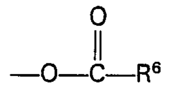

où $R^6$ est un halogénoalkyle ayant jusqu'à 9 atomes de carbone; un cycloalkyle de 3 à 8 atomes de carbone; un alcényle ou un alcynyle ayant jusqu'à 5 atomes de carbone ou

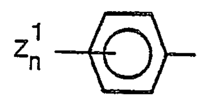

où $Z^1$ est un nitro, un halogène, un trifluorométhyle, un alkyle ou un alcoxy ayant jusqu'à 8 atomes de carbone; et n est 0, 1, 2 ou 3; $R^2$ est un hydrogène, un hydroxy, un alkyle ayant jusqu'à 4 atomes de carbone ou un allyle; et $R^3$ est un alkyle ayant jusqu'à 3 atomes de carbone ou un allyle.

2. Un composé selon la revendication 1, dans lequel R et $R^3$ sont un alkyle, $R^1$ est un hydroxy et $R^2$ est un hydrogène ou un hydroxy.

3. Un composé selon la revendication 2, choisi parmi: la 3 - [5 - (1,1 - diméthyléthyl) - 3 - isoxazolyl] - 1 - méthyl - 4 - hydroxy-2-imidazolidinone, la 3 - [3 - (1,1 - diméthyléthyl) - 5 - isoxazolyl] - 1 - méthyl - 4,5 - dihydroxy - 2 - imidazolidinone, ou la 3 - [5 - (1,1 - diméthyléthyl) - 3 - isoxazolyl] - 1 - méthyl - 4,5 - dihydroxy - 2 - imidazolidone.

4. Un composé selon la revendication 1, où R et $R^3$ sont un alkyle, $R^2$ est un hydrogène et $R^1$ est

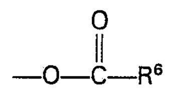

où $R^6$ est un phényle.

5. 3-[5-(1,1-diméthyléthyl)-3-isoxazolyl]-1-méthyl-4-benzoyloxy-2-imidazolidinone.

6. Une composition herbicide contenant une quantité herbicide efficace d'un composé ou d'un mélange de composés comme défini dans la revendication 1.

7. Un procédé de lutte contre la croissance des mauvaises herbes dans lequel on applique une quantité herbicide efficace d'un herbicide à un milieu de croissance avant l'émergence des mauvaises herbes ou on l'applique aux mauvaises herbes après leur émergence du milieu de croissance, caractérisé par le fait que l'herbicide est un composé ou un mélange de composés comme défini dans la revendication 1.

8. Un procédé pour préparer un composé comme revendiqué dans la revendication 1, caractérisé par:

(a) la phosgénation d'un amino-isoxazole convenablement substitué représenté par les formules:

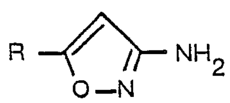

ou

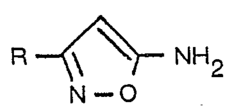

où R est comme défini dans la revendication 1 pour former l'isocyanate correspondant;

(b) la réaction de l'isocyanate avec un amino-acétaldéhyde-dialkylacétal convenablement substitué pour former une acétal-urée de formule:

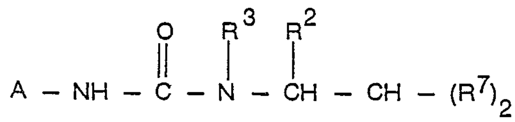

où A et $R^3$ sont comme défini dans la revendication 1, $R^2$ est comme défini dans la revendication 1 à l'exception d'un hydroxy et $R^7$ est un alcoxy ou un alkylthio contenant jusqu'à 6 atomes de carbone; et

(c) l'hydrolyse de l'acétal-urée pour former un composé comme revendiqué dans la revendication 1 où $R^1$ est un hydroxy et $R^2$ est autre qu'un hydroxy.

9. Le procédé de la revendication 8, caractérisé par le stade complémentaire, soit de réaction du produit d'hydrolyse du stade (c) avec un agent d'halogénation pour former un composé revendiqué dans la revendication 1 où $R^1$ est un halogène; soit de réaction du produit d'hydrolyse du stade (c) avec un anhydride de formule:

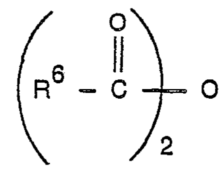

où $R^6$ est comme précédemment défini pour former un composé revendiqué dans la revendication 1 où $R^1$ est

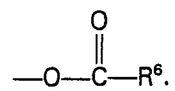

10. Un procédé de préparation d'un composé revendiqué dans la revendication 1, caractérisé par: (a) la réaction d'un amino-isoxazole convenablement substitué représenté par les formules:

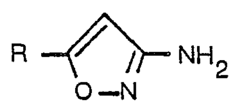 ou 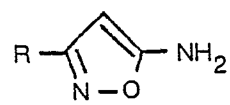

où R est comme défini dans la revendication 1 avec un isocyanate convenablement substitué pour former une urée de formule:

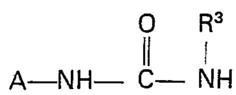

où A et $R^3$ sont comme défini dans la revendication 1; et
(b) la réaction de l'urée avec une solution aqueuse de glyoxal pour former une composé revendiqué dans la revendication 1 où $R^1$ et $R^2$ sont tous deux un hydroxy.

11. Le procédé de la revendication 10, caractérisé par le stade complémentaire, soit de réaction du produit réactionnel du stade (b) avec un agent d'halogénation pour former un composé revendiqué dans la revendication 1 où $R^1$ est un halogène, soit de réaction du produit réactionnel du stade (b) avec un anhydride de formule:

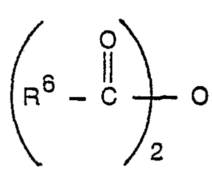

où $R^6$ est comme défini dans la revendication 1 pour former un composé revendiqué dans la revendication 1 où $R^1$ est

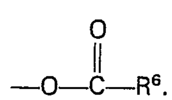

**Revendications pour l'Etat contractant: AT**

1. Procédé de préparation d'un composé ayant la formule:

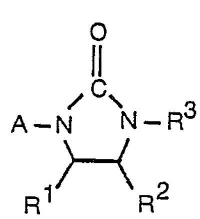

dans laquelle

A est 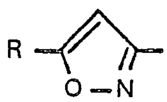 ou 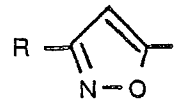

où R est un alkyle ou un halogénoalkyle ayant jusqu'à 6 atomes de carbone; un cycloalkyle de 3 à 8 atomes de carbone; un alcényle ou un alcynyle ayant jusqu'à 5 atomes de carbone; $—R^4—O—R^5$ ou $—R^4—S—R^5$ ou $R^4$ est un alkylène ayant jusqu'à 6 atomes de carbone et $R^5$ est un alkyle ayant jusqu'à 6 atomes de carbone; ou

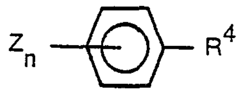 ou 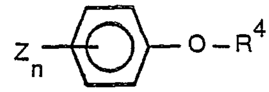

ou Z est un nitro, un halogène, un trifluorométhyle ou $R^5$, et n est 0, 1, 2 ou 3; $R^1$ est un hydroxy, un halogène ou

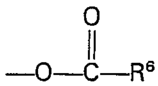

où $R^6$ est un halogénoalkyle ayant jusqu'à 9 atomes de carbone; un cycloalkyle de 3 à 8 atomes de carbone; un alcényle ou un alcynyle ayant jusqu'à 5 atomes de carbone ou

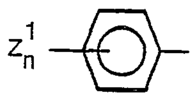

où $Z^1$ est un nitro, un halogène, un trifluorométhyle, un alkyle ou un alcoxy ayant jusqu'à 8 atomes de carbone; et n est 0, 1, 2 ou 3; $R^2$ est un hydrogène, un hydroxy, un alkyle ayant jusqu'à 4 atomes de carbone ou un allyle; et $R^3$ est un alkyle ayant jusqu'à 3 atomes de carbone ou un allyle, caractérisé par:
(a) la phosgénation d'un amino-isoxazole convenablement substitué représenté par les formules:

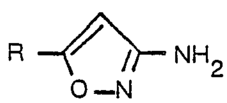 ou 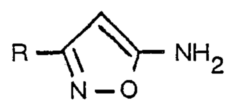

où R est comme défini dans la revendication 1 our former l'isocyanate correspondant;
(b) la réaction de l'isocyanate avec un amino-acétaldéhydedialkylacétal convenablement substitué pour former une acétal-urée de formule:

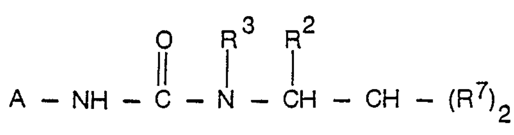

où A et $R^3$ sont comme défini dans la revendication 1, $R^2$ est comme défini dans la revendication 1 à l'exception d'un hydroxy et $R^7$ est alcoxy ou un alkylthio contenant jusqu'à 6 atomes de carbone; et
(c) l'hydrolyse de l'acétal-urée pour former un composé comme revendiqué dans la revendication 1 ou $R^1$ est un hydroxy et $R^2$ est autre qu'un hydroxy.

2. Procédé de la revendication 1, caractérisé par le stade complémentaire, soit de réaction un produit d'hydrolyse du stade (c) avec un agent d'halogénation pour former un composé revendiqué dans la revendication 1 ou $R^1$ est un halogène; soit de réaction du produit d'hydrolyse du stade (c) avec un anhydride de formule:

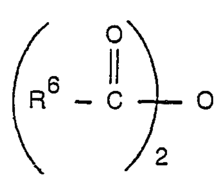

où $R^6$ est comme précédemment défini pour former un composé revendiqué dans la revendication 1 où $R^1$ est

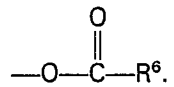

3. Un procédé de préparation d'un composé de formule I, défini dans la revendication 1, caractérisé par:
(a) la réaction d'un amino-isoxazole convenablement substitué représenté par les formules:

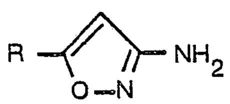 ou 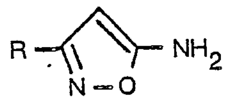

où R est comme défini dans la revendication 1 avec un isocyanate convenablement substitué pour former une urée de formule:

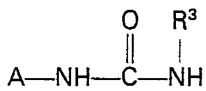

où A et $R^3$ sont comme défini dans la revendication 1; et
(b) la réaction de l'urée avec une solution aqueuse de glyoxal pour former un composé revendiqué dans la revendication 1 où $R^1$ et $R^2$ sont tous deux un hydroxy.

4. Le procédé de la revendication 3, caractérisé par le stade complémentaire, soit de réaction du produit réactionnel du stade (b) avec un agent d'halogénation pour former un composé revendiqué dans la revendication 1 où $R^1$ est un halogène, soit de réaction du produit réactionnel du stade (b) avec un anhydride de formule:

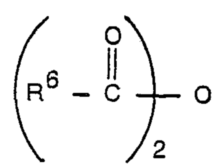

où $R^6$ est comme défini dans la revendication 1 pour former un composé revendiqué dans la revendication 1 où $R^1$ est

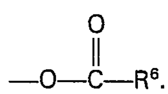

5. Procédé selon la revendication 1, dans lequel R et $R^3$ sont alkyle, $R^1$ est hydroxy et $R^2$ est hydrogène.

6. Procédé selon la revendication 3, dans lequel R et $R^3$ sont alkyle.

7. Procédé selon la revendication 2, dans lequel R et $R^3$ sont alkyle, $R^2$ est hydrogène et $R^1$ est

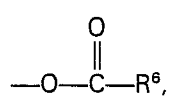

où $R^6$ est phényle.

8. Un procédé de lutte contre le croissance des mauvaises herbes dans lequel on applique un quantité herbicide efficace d'un herbicide à un milieu de croissance avant l'émergence des mauvaises herbes ou on l'applique aux mauvaises herbes après leur émergence du milieu de croissance, caractérisé par le fait que l'herbicide est un composé ou un mélange de composé de formule I comme défini dans la revendication 1.